# EUROPEAN PATENT APPLICATION

(11) **EP 4 696 706 A1**
(43) Date of publication of application: **18.02.2026**
(21) Application number: 24789092.4
(22) Date of filing: 12.04.2024
(51) Int. Cl.: C07K 14/47, C12N 5/074, C12N 15/85, C12N 15/87, A61K 48/00

(54) **FACTOR FOR INDUCING STEMNESS IN CELLS AND METHOD FOR INCREASING OR MAINTAINING STEMNESS IN CELLS BY USING SAME**

(30) Priority: 14.04.2023 KR 20230049466
(71) Applicant: Sungkwang Medical Foundation, Seoul 06135 (KR); Cha University Industry-Academic Cooperation Foundation, Pocheon-si, Gyeonggi-do 11160 (KR)
(72) Inventor: KIM, Min Young, Seoul 06667 (KR); CHOI, Jee In, Seongnam-si, Gyeonggi-do 13500 (KR)
(74) Representative: Vossius & Partner Patentanwälte Rechtsanwälte mbB
(86) International application number: PCT/KR2024/004973
(87) International publication number: WO 2024/215151

(57) **Abstract**

The present specification relates to a factor for inducing stemness in cells and a method of increasing or maintaining stemness in cells by using the same. The factor for inducing stemness in cells and the method of increasing or maintaining stemness in cells by using the same, according to an aspect, have an effect of increasing or maintaining proliferation capacity and stemness in cells, and can be effectively used for increasing therapeutic efficiency and mass culturing, and thus can be used as a cell therapeutic agent. Also, the factor and the method have an effect of exhibiting regenerative efficacy in target tissues upon administration to a living body as a gene therapeutic agent.

## Description

### Technical Field

The present disclosure relates to a factor for inducing stemness in cells and a method of increasing or maintaining stemness in cells by using the same.

### Background Art

Stem cells are cells with multi potency that allow a single cell to produce various types of different cells, and are characterized by renewal of damaged cells in our body. Due to these characteristics, stem cells are emerging as a new alternative for treating degenerative diseases or diseases caused by severe trauma where few other medical options are available. Currently, many stem cell-based therapeutic agents are being developed, and cell reprogramming technologies are being researched using stem cells of various origins.

Embryonic stem cells and induced pluripotent stem cells (iPS) are known as cell therapeutic agents with the highest pluripotency for therapeutic potential. However, in the case of embryonic stem cells, ethical issues have arisen regarding the potential harm to early human life, and accordingly, research is being conducted using induced pluripotent stem cells. In addition, in cell differentiation technology for cell therapy, research results are increasing toward directly differentiating other cells into cells that can exhibit therapeutic effects, rather than obtaining induced pluripotent stem cells and then producing desired cells through subsequent differentiation. A representative example is the induced differentiation of glial cells that increase after brain injury, into non-regenerative central nervous system neurons, where genes that induce partial reprogramming functions are also used.

In addition to cell therapeutic agents, therapeutic attempts through gene introduction for tissue regeneration are being reported. For example, research is increasing in intractable disease models such as neuronal damage, and cases have emerged where regenerative therapy has become possible by delivering a specific gene using adeno-associated virus (AAV) or chemically modified mRNA.

Therefore, there is a need to present a specific method that enables regenerative therapy using genes.

### [Prior Art Documents]

### [Non-Patent Documents]

(Non-Patent Literature 0001) Andrzejewska, Anna, et al. "Mesenchymal stem cells for neurological disorders." Advanced Science 8.7 (2021): 2002944.
(Non-Patent Literature 0002) Hoang, Duc M., et al. "Stem cell-based therapy for human diseases." Signal transduction and targeted therapy 7.1 (2022): 1-41.
(Non-Patent Literature 0003) Han, Yuyi, et al. "The secretion profile of mesenchymal stem cells and potential applications in treating human diseases." Signal Transduction and Targeted Therapy 7.1 (2022): 92.
(Non-Patent Literature 0004) Nabetani, Makoto, Takeo Mukai, and Haruo Shintaku. "Preventing brain damage from hypoxic-ischemic encephalopathy in neonates: update on mesenchymal stromal cells and umbilical cord blood cells." American Journal of Perinatology 39.16 (2022): 1754-1763.
(Non-Patent Literature 0005) Hernandez, A. E., and E. Garcia. "Mesenchymal stem cell therapy for Alzheimer's disease." Stem Cells International 2021 (2021).
(Non-Patent Literature 0006) Chan, Hau Jun, et al. "Therapeutic potential of human stem cell implantation in Alzheimer's disease." International journal of molecular sciences 22.18 (2021): 10151.
(Non-Patent Literature 0007) Si, Zizhen, and Xidi Wang. "Stem cell therapies in Alzheimer's disease: applications for disease modeling." Journal of Pharmacology and Experimental Therapeutics 377.2 (2021): 207-217.
(Non-Patent Literature 0008) Li, Meirong, et al. "Potential pre-activation strategies for improving therapeutic efficacy of mesenchymal stem cells: current status and future prospects." Stem cell research & therapy 13.1 (2022): 146.
(Non-Patent Literature 0009) Paolini Sguazzi, Giulia, et al. "Induced pluripotent stem cells (iPSCs) and gene therapy: a new era for the treatment of neurological diseases." International Journal of Molecular Sciences 22.24 (2021): 13674.
(Non-Patent Literature 0010) Chehelgerdi, Matin, et al. "Exploring the promising potential of induced pluripotent stem cells in cancer research and therapy." Molecular Cancer 22.1 (2023): 189.

### Disclosure of Invention

### Technical Problem

An aspect is to provide a nucleic acid molecule including a nucleotide sequence encoding an amino acid sequence having at least 90 % sequence identity with the amino acid sequence of SEQ ID NO: 1, or a vector including the nucleic acid molecule.

Another aspect is to provide a factor for inducing stemness in cells, the factor including a nucleotide sequence encoding an amino acid sequence having at least 90 % sequence identity with the amino acid sequence of SEQ ID NO: 1.

Another aspect is to provide use of the factor for inducing stemness in cells for inducing stemness in cells.

Another aspect is to provide cells having increased or maintained stemness by the factor for inducing stemness in cells.

Another aspect is to provide a composition for increasing or maintaining stemness in cells, including one or more selected from the group consisting of the factor for inducing stemness in cells, a vector including the factor, and a protein of the factor.

Another aspect is to provide a method of increasing or maintaining stemness in cells, including treating the cells with the composition.

Another aspect is to provide a method of producing stem cells having increased or maintained stemness, including treating cells with the composition.

Another aspect is to provide a method of producing stem cells having increased or maintained stemness, including bringing cells into contact with the factor for inducing stemness in cells.

Another aspect is to provide a method of increasing or maintaining differentiation capacity, proliferation capacity and/or stemness in cells, including bringing the cells into contact with the factor for inducing stemness in cells.

Another aspect is to provide stem cells obtained by the method.

Another aspect is to provide somatic cells derived from the stem cells through induced differentiation.

Another aspect is to provide a method of preventing or treating a disease, including administering one or more selected from the group consisting of the factor for inducing stemness in cells, a vector including the factor, and a protein of the factor, to a subject in need thereof.

Another aspect is to provide a gene therapeutic composition including one or more selected from the group consisting of the factor for inducing stemness in cells and a vector including the factor.

Another aspect is to provide a cell therapeutic composition including cells which include one or more selected from the group consisting of the factor for inducing stemness in cells, a vector including the factor, and a protein of the factor.

Another aspect is to provide an extracellular vesicle therapeutic composition including exosomes secreted by the cells.

Another aspect is to provide use of one or more selected from the group consisting of the factor for inducing stemness in cells and a vector including the factor for gene therapy.

Another aspect is to provide use of cells for cell therapy, the cells including one or more selected from the group consisting of the factor for inducing stemness in cells, a vector including the factor, and a protein of the factor.

Another aspect is to provide use of exosomes secreted by the cells for extracellular vesicle therapy.

Another aspect is to provide use for preparing a gene therapeutic agent, including one or more selected from the group consisting of the factor for inducing stemness in cells and a vector including the factor.

Another aspect is to provide use for preparing a cell therapeutic agent, including cells which include one or more selected from the group consisting of the factor for inducing stemness in cells, a vector including the factor, and a protein of the factor.

Another aspect is to provide use for preparing an extracellular vesicle therapeutic agent including exosomes secreted by the cells.

Another aspect is to provide a delivery vehicle (e.g., mRNA, exosomes, liposomes, or a biocompatible polymer) including the factor for inducing stemness in cells.

### Solution to Problem

An aspect provides a nucleic acid molecule including a nucleotide sequence encoding an amino acid sequence having at least 90 % sequence identity with the amino acid sequence of SEQ ID NO: 1, or a vector including the nucleic acid molecule.

Another aspect provides a factor for inducing stemness in cells, the factor including a nucleotide sequence encoding an amino acid sequence having at least 90 % sequence identity with the amino acid sequence of SEQ ID NO: 1.

The factor for inducing stemness in cells may be a gene or a polynucleotide.

In an embodiment, the nucleotide sequence may be a nucleotide sequence encoding an amino acid sequence having at least 80 %, 85 %, 90 %, 95 %, 96 %, 97 %, 98 % or 99 % sequence identity with the amino acid sequence of SEQ ID NO: 1.

In an embodiment, the factor for inducing stemness in cells may include a nucleotide sequence encoding the amino acid sequence of SEQ ID NO: 1.

The nucleotide sequence encoding the amino acid sequence of SEQ ID NO: 1 may be represented by SEQ ID NO: 2.

In an embodiment, the nucleotide sequence may be a nucleotide sequence having at least 80 %, 85 %, 90 %, 95 %, 96 %, 97 %, 98 %, or 99 % sequence identity with the nucleotide sequence represented by SEQ ID NO: 2.

Another aspect provides use of the factor for inducing stemness in cells for inducing stemness in cells.

In an embodiment, the nucleic acid molecule may have a portion of the nucleotide sequence represented by SEQ ID NO: 2. The nucleic acid molecule may include a nucleotide sequence having at least 80 %, 85 %, 90 %, 95 %, 96 %, 97 %, 98 % or 99 % sequence identity with the nucleotide sequence represented by SEQ ID NO: 1.

It is obvious that amino acid sequences or nucleotide sequences exhibiting such sequence identity or identity, in which some sequences are deleted, modified, substituted, conservatively substituted, or added, also fall within the scope of the present application.

The term "sequence identity" or "identity" as used herein refers to the degree to which two given amino acid sequences or nucleotide sequences are related, which may be expressed as a percentage. The terms "sequence identity" and "identity" may often be used interchangeably.

In the present disclosure, SEQ ID NO. 3 represents the amino acid sequence of human pericentrin (PCNT).

The amino acid sequence of SEQ ID NO: 1 represents a partial sequence of the amino acid sequence of human PCNT of SEQ ID NO: 3.

A protein including the amino acid sequence of SEQ ID NO: 1 may be regarded as a new material different from human PCNT. This is because the protein MK145 having the amino acid sequence of SEQ ID NO: 1 of the present disclosure corresponds to approximately 4.3 % of the total size of a PCNT protein, and thus the structures of the proteins are different from each other, and accordingly, cannot be considered to have the same function. Therefore, the protein including the amino acid sequence of SEQ ID NO: 1 of the present disclosure consists of an amino acid sequence identical to a portion of a PCNT sequence, but is a newly discovered and defined substance.

The nucleotide sequence of SEQ ID NO: 2 may be, but is not limited to, a nucleotide sequence encoding a fragment of the PCNT protein.

The term "vector" as used herein may refer to a polynucleotide construct containing the regulatory sequence and nucleotide sequence of a gene so as to express a gene of interest in a suitable host cell. The vector may also refer to a polynucleotide construct containing a nucleotide sequence capable of homologous recombination that, when introduced into a host cell, can change a regulatory sequence of an endogenous gene on the genome of a host or insert, into a specific genomic site, a gene of interest that can be expressed. Therefore, the vector may further include a selection marker to confirm whether the vector is introduced into a host or inserted into the chromosome, and the selection marker may include markers that impart selectable phenotypes such as drug resistance, auxotrophy, resistance to cytotoxic agents, or surface protein expression. In an environment treated with a selective agent, only cells expressing the selection marker survive or different phenotypic traits are exhibited, thereby enabling the selection of transformed cells. For example, the vector may be, but is not limited to, a plasmid vector, a cosmid vector, a viral vector, a lentivirus vector, a retrovirus vector, a human immunodeficiency virus (HIV) vector, a murine leukemia virus (MLV) vector, an avian sarcoma/leukosis (ASLV) vector, a spleen necrosis virus (SNV) vector, a Rous sarcoma virus (RSV) vector, a mouse mammary tumor virus (MMTV) vector, an adenovirus vector, an adeno-associated virus vector, a herpes simplex virus vector, and an episomal vector. In other embodiments, vectors that may be used as the recombinant vector may be constructed by manipulating plasmids commonly used in the art (e.g., pSC101, pGV1106, pACYC177, ColE1, pKT230, pME290, pBR322, pUC8/9, pUC6, pBD9, pHC79, plJ61, pLAFR1, pHV14, pGEX series, pET series, and pUC19), phages (e.g., λgt4λB, λ-Charon, λΔz1, and M13), or viruses (e.g., SV40). Preferably, pET28a plasmid vector (Novagen, Germany) may be used as the vector.

In a recombinant vector into which the nucleic acid molecule is introduced, the nucleic acid molecule may be operably linked to a promoter. The term "operably linked" means that a promoter sequence that initiates and mediates transcription of a polynucleotide encoding a gene of interest is operably linked to the polynucleotide sequence.

The recombinant vector may typically be constructed as a cloning vector or an expression vector. The expression vector may be those commonly used to express foreign proteins in plants, animals, or microorganisms in the art. The recombinant vector may be constructed by various methods known in the art.

The recombinant vector may be constructed using a prokaryotic cell or eukaryotic cell as a host. For example, in case that an expression vector is used and a prokaryotic cell is used as a host, the vector generally includes strong promoters capable of driving transcription (e.g., a pLλ promoter, a CMV promoter, a trp promoter, a lac promoter, a tac promoter, and a T7 promoter), a ribosome binding site for initiation of translation, and a transcription/translation termination sequence. In the case of using a eukaryotic cell as a host, origins of replication that function in eukaryotic cells included in the vector include, but are not limited to, an f1 origin of replication, a SV40 origin of replication, a pMB1 origin of replication, an adeno origin of replication, an AAV origin of replication, and a BBV origin of replication. In other embodiments, promoters derived from the genomes of mammalian cells (e.g., metallothionein promoter) or promoters derived from mammalian viruses (e.g., adenovirus late promoter, vaccinia virus 7.5K promoter, SV40 promoter, cytomegalovirus promoter, and tk promoter of HSV) may be used, and the transcription termination sequence is generally a polyadenylation sequence.

The recombinant vector may be obtained by treating a nucleic acid molecule and a plasmid vector with the same restriction enzyme, and then introducing the nucleic acid molecule treated with the restriction enzyme into the plasmid vector treated with the same restriction enzyme.

In an embodiment, the restriction enzyme may be Nhel, BamHI, EcoRI, Hindlll, kpnI, Notl, Pstl, Smal, Xhol, Fokl, Alw26I, Bbvl, Bsrl, Earl, Hphl, Mbol, SfaNI, Tth1111, Neal, NgoMIV, Eco57I, Bcgl, Bpl, Bsp24I, Bael, CjeI, EcoPI, StyLTI, Bal31, T4, T7, RecBCD, P25736I, P09030II, P1, Dnasel, Endo R, Cas9, Cpf1, C2c1, C2c2, C2c3, Cas3, Cas3-HD, Cas5, Cas7, Cas8, and Cas10. Preferably, the restriction enzyme may be, but is not limited to, Nhel or BamHI.

In an embodiment, the cells may be somatic cells, lineage-restricted progenitor cells, or stem cells.

The term "somatic cells" may refer to all cells excluding reproductive cells, and somatic cells may be derived or isolated from, for example, mammals such as humans, monkeys, cows, horses, sheep, pigs, goats, camels, antelopes, dogs, cats, mice, and rabbits, but the disclosure is not limited thereto. Preferably, the somatic cells may be isolated from a human.

The somatic cells may be fibroblasts, epithelial cells, muscle cells, cardiac cells, kidney cells, neurons, hair cells, hair root cells, hair follicle cells, oral epithelial cells, beta cells, gastric mucosal cells, goblet cells, G cells, B cells, pericytes, hyalocytes, odontoblasts, chondrocytes, or cells extracted from urine, saliva, sweat, or blood from the human body.

The lineage-restricted progenitor cells may be any one selected from the group consisting of neural progenitor cells, glial progenitor cells, blood endothelial progenitor cells, mesenchymal progenitor cells, hematopoietic progenitor cells, pancreatic progenitor cells, myeloid progenitor cells, and lymphoid progenitor cells.

The term "stem cells" as used herein refers to undifferentiated cells that have the ability to self-replicate and differentiate into two or more different types of cells. The stem cells may be totipotent stem cells, pluripotent stem cells, or multipotent stem cells.

In an embodiment, the stem cells may be embryonic stem cells, adult stem cells, or induced pluripotent stem cells.

The term "adult stem cells" as used herein refers to relatively undifferentiated cells located between differentiated cells in tissues and organs. Adult stem cells proliferate through somatic cell division to regulate their number and are responsible for functions such as cellular replacement, functional recovery, and tissue remodeling in tissues where these cells reside. Adult stem cells are named according to the organ where these cells are found, and capabilities thereof are also limited to some extent.

The adult stem cells may be derived from one or more tissues selected from the group consisting of umbilical cord blood, adipose, nerve, bone marrow, peripheral blood, muscle, liver, skin, placenta, amniotic membrane, embryo, and umbilical cord. Preferably, the adult stem cells may be derived from adipose tissue or embryo.

The adult stem cells may be one or more types selected from the group consisting of hematopoietic stem cells (HSCs), mesenchymal stem cells (MSCs), neural stem cells (NSCs), cardiac stem cells (CSCs), intestinal stem cells (ISCs), muscle stem cells (MuSCs), follicle stem cells (FSCs), and endothelial progenitor cells (EPCs).

The term "mesenchymal stem cells (MSCs)" as used herein may refer to stem cells present in cartilage, bone tissue, adipose tissue, bone marrow stroma, and the like, which are differentiated from the mesoderm generated by division of a fertilized egg, and may include mesenchymal stem cells of mammals, for example, animals including humans. Mesenchymal stem cells may have the ability to maintain stemness and self-renewal and to differentiate into various cells, including chondrocytes, osteoblasts, muscle cells, and adipocytes. The mesenchymal stem cells may be derived from one or more selected from the group consisting of embryo, bone marrow, adipose tissue, umbilical cord blood, synovial membrane, trabecular bone, muscle, neural tissue, and infrapatellar fat pad. Mesenchymal stem cells have immunomodulatory abilities that suppress the activity and proliferation of T lymphocytes and B lymphocytes, inhibit the activity of natural killer cells (NK cells), and regulate the functions of dendritic cells and macrophages, making them suitable for allotransplantation and xenotransplantation.

In an embodiment, the adult stem cells may be, but are not limited to, embryonic stem cell-derived mesenchymal stem cells (ES-MSCs) and adipose-derived mesenchymal stem cells (AD-MSCs).

The term "induced pluripotent stem cells (iPSCs)" as used herein are also referred to as induced pluripotent stem cells, and refers to cells having pluripotency obtained by dedifferentiation from differentiated cells (e.g., somatic cells). In this specification, induced pluripotent stem cells may be used to mean not only fully induced pluripotent stem cells, but also partially induced pluripotent stem cells, induced progenitor cells or stem cells, or other types of partially differentiated cells.

The induced pluripotent stem cells may differentiate into various organ cells. The induced pluripotent stem cells may be obtained by reprogramming differentiated cells by using reprogramming factors.

The term "differentiation" as used herein refers to a phenomenon in which cells become specialized in structure or function during division, proliferation, and growth, i.e., a process by which biological cells, tissues, and the like change in form or function to perform their assigned tasks.

The term "dedifferentiation" as used herein refers to the opposite of differentiation, which is a reversible phenomenon in which already fully differentiated cells change their characteristics to an early undifferentiated state through epigenetic changes.

The term "differentiated cells" as used herein refers to cells that have proceeded further downstream the developmental pathway than the cells being considered. Therefore, stem cells may differentiate into lineage-restricted progenitor cells (e.g., myocyte progenitor cells), which can eventually differentiate further downstream the pathway into other types of progenitor cells (e.g., myocyte progenitors), and subsequently into terminally differentiated cells, such as myocytes, which perform characteristic roles in certain tissue types and may or may not retain the ability to proliferate further.

The term "dedifferentiated cells" as used herein refers to cells that have proceeded further upstream the developmental pathway than the cells being considered. Thus, lineage-restricted progenitor cells may dedifferentiate into stem cells, and somatic cells may dedifferentiate into lineage-restricted progenitor cells.

In an embodiment, the factor for inducing stemness in cells may increase or maintain proliferation capacity and/or stemness in cells.

The term "growth" or "proliferation" as used herein refers to an increase in the number of cells. The proliferation capacity may refer to self-proliferation capacity that allows stem cells to proliferate indefinitely for a long period of time.

The term "stemness" as used herein may refer to self-renewal capacity, which enables continuous self-replication, and pluripotency, which enables differentiation into various types of cells.

In this specification, inducing stemness may include the concept of inducing dedifferentiation.

The stemness may be confirmed through the expression of the factor for inducing stemness, which consists of Nanog, octamer-binding transcription factor 4 (OCT4), sex determining region Y (SRY)-box 2 (SOX-2), and Kruppel-like factor 4 (KIf4). The increase or maintenance of the stemness may include increasing or maintaining the expression of one or more of factors for inducing stemness. Therefore, the factor for inducing stemness in cells may increase or maintain the expression or activity of one or more selected from the group consisting of Nanog, OCT4, SOX-2, and Klf4.

The increase or maintenance of the expression of the factor for inducing stemness in cells may result in improved or maintained culture yield and superior characteristics of stem cells.

Another aspect provides cells having increased or maintained stemness by the factor for inducing stemness.

Another aspect provides a composition for increasing or maintaining stemness in cells, the composition including one or more selected from the group consisting of: the factor for inducing stemness in cells; a vector including the factor; and a protein of the factor.

The vector may be one or more vectors selected from the group consisting of a plasmid vector, a cosmid vector, a viral vector, a lentivirus vector, a retrovirus vector, a human immunodeficiency virus (HIV) vector, a murine leukemia virus (MLV) vector, an avian sarcoma/leukosis (ASLV) vector, a spleen necrosis virus (SNV) vector, a Rous sarcoma virus (RSV) vector, a mouse mammary tumor virus (MMTV) vector, an adenovirus vector, an adeno-associated virus vector, a Herpes simplex virus vector, and an episomal vector.

In an embodiment, the composition may cause: increased proliferation capacity of cells; induction of dedifferentiation of cells; or both.

In an embodiment, the composition may increase the expression or activity of one or more selected from the group consisting of Nanog, octamer-binding transcription factor 4 (OCT4), sex determining region Y (SRY)-box 2 (SOX-2), and Kruppel-like factor 4 (Klf4).

Another aspect provides a method of increasing or maintaining stemness in cells, the method including treating the cells with the composition for increasing or maintaining stemness in cells.

Another aspect provides a method of producing stem cells having increased or maintained stemness, the method including treating cells with the composition for increasing or maintaining stemness in cells.

In an embodiment, dedifferentiation of cells may be induced by the increased stemness.

Another aspect provides a method of producing stem cells having increased or maintained stemness, including bringing cells into contact with the factor for inducing stemness in cells.

Another aspect provides a method of increasing or maintaining differentiation capacity, proliferation capacity and/or stemness in cells, the method including bringing the cells into contact with the factor for inducing stemness in cells.

Another aspect provides stem cells obtained by the method.

Another aspect provides somatic cells derived from the stem cells through induced differentiation.

In an embodiment, the bringing of the cells into contact may include delivering or introducing the factor for inducing stemness in cells to the cells. For the delivery or introduction, a method of providing a nucleic acid molecule to cells, which is commonly used in the art, may be used without limitation. Preferably, a method of administration to a cell culture medium or a method of direct injection into cells may be used. Any method known in the art may be selected and used as a method of directly injecting the nucleic acid molecule into differentiated cells, and may be appropriately selected and applied from, but not limited to, microinjection, electroporation, particle bombardment, direct muscle injection, and methods using an insulator and a transposon.

In an embodiment, lipofectamine may be used to deliver or introduce the factor for inducing stemness in cells into the cells, but the disclosure is not limited thereto.

To deliver or introduce the factor for inducing stemness to cells, naked DNA in vector form may be introduced into cells (Wolff et al. Science, 1990: Wolffet al. J Cell Sci. 103:1249-59, 1992), or liposomes, a cationic polymer, mRNA, exosomes, or the like may be introduced into cells.

The term "exosomes" as used herein refers to cell-derived vesicles that exist in the body fluids of almost all eukaryotic organisms and have a diameter of about 30-100 nm, which are larger than LDL proteins but much smaller than erythrocytes. Exosomes are well known to be released from cells when multivesicular bodies fuse with the cell membrane or to be released directly from the cell membrane, and to perform important and specialized functions such as coagulation and intercellular signaling.

Another aspect provides a method of preventing or treating a disease, the method including administering one or more selected from the group consisting of: the factor for inducing stemness in cells; a vector including the factor; and a protein of the factor to a subject in need thereof.

In an embodiment, the disease may be a neurological disease.

In an embodiment, the neurological disease may include all diseases in which an abnormality or disorder occurs in some or all of the brain, spinal cord, cranial nerves, spinal nerves, and autonomic nervous system that constitute the nervous system.

In an embodiment, the neurological disease may be one or more selected from the group consisting of ischemic stroke, hemorrhagic stroke, ischemic brain injury, traumatic brain injury, cerebral palsy, mental disorder, and neurodegenerative disease.

The mental disorder may be one or more selected from the group consisting of autism spectrum disorder, schizophrenia, intellectual disability, and Down syndrome.

The neurodegenerative disease may be, but is not limited to, one or more selected from the group consisting of dementia, vascular dementia, Alzheimer's disease, Parkinson's disease, Huntington's disease, mild cognitive impairment, cerebral amyloid angiopathy, amyloid stroke, systemic amyloid disease, Dutch amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

There are various research papers on the treatment of neurological diseases using stem cells and their mechanisms of action (see Non-Patent Literature 1, Non-Patent Literature 2, Non-Patent Literature 3, and Non-Patent Literature 4). There are also various research papers on the treatment of neurodegenerative disease using stem cells and their mechanisms of action (see Non-Patent Literature 5, Non-Patent Literature 6, and Non-Patent Literature 7). Non-Patent Literature 8 also discloses that therapeutic effects of diseases can be enhanced when stemness is increased. Non-Patent Literature 9 also discloses a method of treating a neurological disease by using induced pluripotent stem cells (iPSCs) in combination with gene therapy. Non-Patent Literature 10 also discloses the applicability of induced pluripotent stem cells as a cell therapeutic agent.

In view of Non-Patent Literature 1 to 10 and examples and experimental examples of the disclosure, which will be described below, a factor for inducing stemness in cells, a vector including the factor, a protein of the factor, or cells including one or more thereof, according to an aspect of the present disclosure, has an effect of preventing or treating a neurological disease or a neurodegenerative disease, and has an effect of increasing or maintaining a preventive or therapeutic effect on a neurological disease or a neurodegenerative disease by increasing or maintaining stemness.

Another aspect provides a gene therapeutic composition including one or more selected from the group consisting of: the factor for inducing stemness in cells; and a vector including the factor.

Another aspect provides a cell therapeutic composition including cells which include one or more selected from the group consisting of: the factor for inducing stemness in cells; a vector including the factor; and a protein of the factor.

Another aspect provides an extracellular vesicle therapeutic composition including exosomes secreted by the cells.

In an embodiment, the gene therapeutic composition, the cell therapeutic composition, or the extracellular vesicle therapeutic composition may be a gene therapeutic composition, a cell therapeutic composition, or an extracellular vesicle therapeutic composition, for the prevention or treatment of a neurological disease.

The neurological disease associated with the composition is as described in relation to the method of preventing or treating a neurological disease.

Another aspect provides use of one or more selected from the group consisting of: the factor for inducing stemness in cells; and a vector including the factor, for gene therapy.

Another aspect provides use of cells for cell therapy, the cells including one or more selected from the group consisting of: the factor for inducing stemness in cells; a vector including the factor; and a protein of the factor.

Another aspect provides use of exosomes secreted by the cells for extracellular vesicle therapy.

In an embodiment, the use for gene therapy, cell therapy, or extracellular vesicle therapy may be use for gene therapy, cell therapy, or extracellular vesicle therapy of a neurological disease.

The neurological disease associated with the use for therapy is as described in relation to the method of preventing or treating a neurological disease.

Another aspect provides use for preparing a gene therapeutic agent, including one or more selected from the group consisting of: the factor for inducing stemness in cells; and a vector including the factor.

Another aspect provides use for preparing a cell therapeutic agent, including cells which include one or more selected from the group consisting of: the factor for inducing stemness in cells; a vector including the factor; and a protein of the factor.

Another aspect provides use for preparing an extracellular vesicle therapeutic agent including exosomes secreted by the cells.

In an embodiment, the use for preparing a gene therapeutic agent, a cell therapeutic agent, or an extracellular vesicle therapeutic agent may be use for preparing a gene therapeutic agent, a cell therapeutic agent, or an extracellular vesicle therapeutic agent, for the prevention or treatment of a neurological disease.

The neurological disease associated with the use for preparing a therapeutic agent is as described in relation to the method of preventing or treating a neurological disease.

Another aspect provides a delivery vehicle (e.g., mRNA, exosomes, liposomes, or a biocompatible polymer) including the factor for inducing stemness in cells.

In an embodiment, the vector of the present disclosure may include one or more selected from the group consisting of mRNA, exosomes, liposomes, and biocompatible polymers.

The term "cell therapeutic agent" as used herein refers to a therapeutic agent used to treat a disease by using autologous, allogenic, or xenogenic cells to restore tissue functions.

The term "gene therapeutic agent" as used herein refers to a therapeutic agent that uses a gene to treat or prevent a disease. Treatment can be achieved by a method of inserting a new gene into cells of a patient, removing malfunctioning genes, or replacing a mutated gene with a normal gene. For gene delivery, a lentivirus vector or a physical method such as electroporation, or an adenovirus-associated virus (AAV) vector and lipid nanoparticles may be used as delivery vehicles, but the disclosure is not limited thereto, and various methods known in the art may be used to construct delivery vehicles.

The term "extracellular vesicle therapeutic agent" as used herein refers to a therapeutic agent prepared by isolating and purifying extracellular vesicles secreted from living cells. Extracellular vesicles may include exosomes, microvesicles, and the like, and may be administered via various routes without the risk of embolism, which is a potential risk factor for a cell therapeutic agent. To obtain the extracellular vesicles, ultrafiltration, ultracentrifugation, precipitation, chromatography, and a combination of one or more of these methods may be used.

The cell therapeutic agent, the gene therapeutic agent, or the extracellular vesicle therapeutic agent may further include a pharmaceutically acceptable carrier. The pharmaceutically acceptable carrier may be, for example, saline, sterile water, Ringer's solution, buffered saline, dextrose solution, maltodextrin solution, glycerol, ethanol, human serum albumin (HSA), and a mixture of one or more of these components. If necessary, other conventional additives such as antioxidants, buffers, and bacteriostatic agents may be added. In other embodiments, to enable specific action on target organs, target organ-specific antibodies or other ligands may be used in combination with the carrier.

The cell therapeutic agent, the gene therapeutic agent, or the extracellular vesicle therapeutic agent may further include, for example, a diluent, a dispersant, a surfactant, a binder, and a lubricant, and for example, may be formulated into an injectable formulation such as an aqueous solution, a suspension, or an emulsion.

In an embodiment, the dosage of cells may be 1.0 x 10⁵ to 1.0 x 10⁸ cells/kg (body weight). However, the dosage may be prescribed in various ways depending on factors such as the formulation method, the administration method, the age, body weight and gender of a patient, pathological condition, diet, administration time, administration route, excretion rate, and response sensitivity, and may be appropriately adjusted by those of ordinary skill in the art considering these factors. The number of administrations may be once or twice or more within the range of clinically acceptable side effects, and an administration site may be one or more sites. For animals other than humans, the same dosage per kg as for humans may be administered, or an amount calculated by converting the dosage according to, for example, the volume ratio (e.g., average value) of organs (such as the heart) between a target animal and a human may be administered. Possible routes of administration may include oral, sublingual, parenteral (e.g., subcutaneous, intramuscular, intraarterial, intraperitoneal, intrathecal, or intravenous), rectal, topical (including transdermal), inhalation, and injection, or insertion of an implantable devices or materials.

Examples of animals to be treated according to an embodiment may include humans and other target mammals, and specific examples thereof include humans, monkeys, mice, rats, rabbits, sheep, cows, dogs, horses, pigs, and the like.

### Advantageous Effects of Invention

A factor for inducing stemness in cells and a method of increasing or maintaining stemness in cells by using the same, according to an aspect, have an effect of increasing or maintaining proliferation capacity and stemness in cells, and can be effectively used for increasing therapeutic efficiency and mass culturing, and thus can be used as a cell therapeutic agent and a gene therapeutic agent.

### Brief Description of Drawings

FIG. 1 is a graph confirming an optimal recombinant vector for inducing stemness that overexpresses a gene including the nucleotide sequence of SEQ ID NO: 1.
FIG. 2 is a graph confirming gene expression by RT-PCR in stem cells transduced with a recombinant plasmid vector.
FIG. 3 confirms an increase in the number of cells in ES-MSCs transduced with a recombinant plasmid vector.
FIG. 4 confirms an increase in cell growth in ES-MSCs transduced with a recombinant plasmid vector.
FIG. 5 illustrates graphs confirming the expression of stemness markers by RT-PCR in ES-MSCs transduced with a recombinant plasmid vector.
FIG. 6 confirms an increase in the number of cells in AD-MSCs transduced with a recombinant plasmid vector.
FIG. 7 illustrates graphs confirming the expression of stemness markers by RT-PCR in AD-MSCs transduced with a recombinant plasmid vector.
FIG. 8 confirms an increase in the number of cells in a fibroblast cell line transfected with a recombinant plasmid vector.
FIG. 9 is a graph confirming the expression of KIf4, which is a stemness marker, in a fibroblast cell line transduced with a recombinant plasmid vector.
FIG. 10 shows the mNSS test results on days 1, 3, 7, 14, 21, and 28 upon treatment with ES-MSCs and ES-MSC+MK145 in a stroke animal model.
FIG. 11 shows improved behavioral ability scores from day 7 (administration day of ES-MSCs and ES-MSC+MK145) to day 28 in a stroke animal model.
FIG. 12 shows the Western blot results of neurogenesis markers upon treatment with ES-MSCs and ES-MSC+MK145 in a stroke animal model.
FIG. 13 shows the Western blot results of angiogenesis markers upon treatment with ES-MSCs and ES-MSC+MK145 in a stroke animal model.
FIG. 14 shows the treatment schedule in a stroke cell model (OGD model).
FIG. 15 shows the results of cell recovery and proliferation analysis upon treatment with ES-MSCs and ES-MSC+MK145 in a stroke cell model.
FIG. 16 shows the treatment schedule in an amyloid beta injection animal model.
FIG. 17 shows the results of analyzing behavioral/cognitive ability in a novel object recognition test (NORT) upon treatment with ES-MSCs and ES-MSC+MK145 in an amyloid beta injection animal model.
FIG. 18 shows the results of analyzing behavioral/cognitive ability in a passive avoidance test upon treatment with ES-MSCs and ES-MSC+MK145 in an amyloid beta injection animal model.
FIG. 19 shows the treatment schedule in an APP/PS1 TG animal model.
FIG. 20 shows the results of analyzing behavioral/cognitive ability in a novel object recognition test (NORT) upon treatment with ES-MSCs and ES-MSC+MK145 in an APP/PS1 TG animal model.

### Mode for the Invention

Hereinafter, preferred examples will be presented to aid in understanding of the present disclosure. However, these examples are provided only to facilitate the understanding of the present disclosure and are not intended to limit the scope of the present disclosure. Embodiments may allow for various modifications, and thus are not limited by the examples disclosed below and may be embodied in various forms.

### Example 1. Production of cells including recombinant plasmid vector

### 1.1. Construction of recombinant plasmid vector

A recombinant plasmid vector into which was introduced a nucleic acid molecule including the nucleotide sequence of SEQ ID NO: 2 was constructed using the pET28a plasmid vector (Novagen, Germany). Hereinafter, the nucleotide sequence of SEQ ID NO: 2 is also referred to as "MK145."

Specifically, a primer pair was designed to induce the expression of a gene including MK145, and polymerase chain reaction was performed. The reaction product was digested with restriction enzymes (*Nhe*I*, Bam*HI), and then cloned into the pET28a plasmid vector to produce a pET28a-MK145 recombinant plasmid into which the nucleic acid molecule was introduced.

### 1.2. Confirmation of optimal plasmid vector conditions for gene overexpression

To confirm plasmid sequence conditions for overexpression of a gene including the nucleotide sequence of SEQ ID NO: 2 in somatic cells, various sizes of DNA were transduced into somatic cells for comparative analysis.

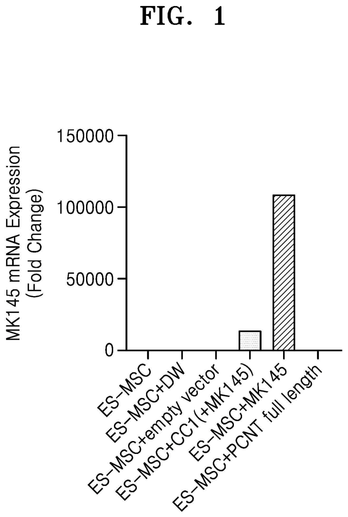

A vector into which only the nucleotide sequence of SEQ ID NO: 2 was introduced, a vector into which was introduced CC1 (a fragment of PCNT) including the nucleotide sequence of SEQ ID NO: 2, or a vector into which PCNT was introduced was used, and distilled water and an empty vector were used together for comparison. The vectors were transfected into embryonic stem cell-derived mesenchymal stem cells (ES-MSCs) using Lipofectamine reagent, and the transfected cells were cultured for 24 hours. After 24 hours, RT-PCR was performed to confirm the mRNA expression level of the gene including the nucleotide sequence of SEQ ID NO: 2. The sequences of primers used in the RT-PCR are shown in Table 1, and the mRNA expression levels through RT-PCR are shown in FIG. 1 and Table 2.

**Table 1**

| **Name** | **Primer** | **Sequence (3'→5')** |
|---|---|---|
| Human MK145 | Forward | GCTGAACGGGCCCTTAGGA (SEQ ID NO: 4) |
| | Reverse | CCTGTCTACTGGTCCTGGCA (SEQ ID NO: 5) |
| Human 18s rRNA | Forward | GTAACCCGTTGAACCCCATT (SEQ ID NO: 6) |
| | Reverse | CCATCCAATCGGTAGTAGCG (SEQ ID NO: 7) |

**Table 2**

| | ES-MSC | ES-MSC +DW | ES-MSC + empty vector | ES-MSC +CC1 | ES-MSC +MK145 | ES-MSC +PCNT full length |
|---|---|---|---|---|---|---|
| MK145 mRNA Expression (Fold change) | 1 | 1.3 | 1.4 | 13587.6 | 108324.5 | 1341.8 |

FIG. 1 is a graph confirming an optimal recombinant vector for inducing stemness that overexpresses a gene including the nucleotide sequence of SEQ ID NO: 2.

As shown in FIG. 1, it was confirmed that mRNA expression was highest in an ES-MSC cell line transduced with a vector into which the nucleotide sequence of SEQ ID NO: 2 was introduced alone compared to vectors into which CC1 or PCNT was introduced.

### 1.3. Confirmation of gene overexpression in various cells

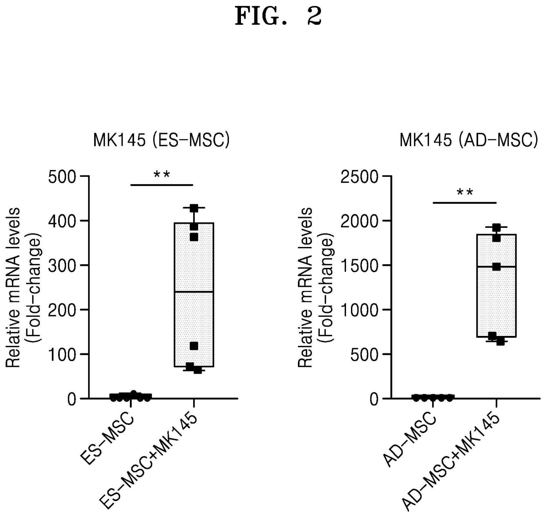

To confirm gene overexpression through transfection in various cell lines, embryonic stem cell-derived mesenchymal stem cells (ES-MSCs) and adipose-derived mesenchymal stem cells (AD-MSCs) were transfected with the pET28a-MK145 recombinant plasmid produced by the method of Example 1.1. After 24 hours, cells were harvested and the level of gene overexpression was confirmed by RT-PCR. The results thereof are shown in FIG. 2.

FIG. 2 is a graph confirming gene expression by RT-PCR in stem cells transduced with a recombinant plasmid vector.

As shown in FIG. 2, gene overexpression was confirmed not only in ES-MSCs transduced with the recombinant plasmid vector but also in the AD-MSC cell line transduced with the recombinant plasmid vector.

From the above results, it was confirmed that a gene including the nucleotide sequence of SEQ ID NO: 2 was overexpressed when somatic cells were transduced with the recombinant plasmid vector into which the nucleotide sequence of SEQ ID NO: 2 was introduced.

### Experimental Example 1. Confirmation of increased stemness in ES-MSCs transduced with recombinant plasmid

### Confirmation of increased cell proliferation capacity in ES-MSCs

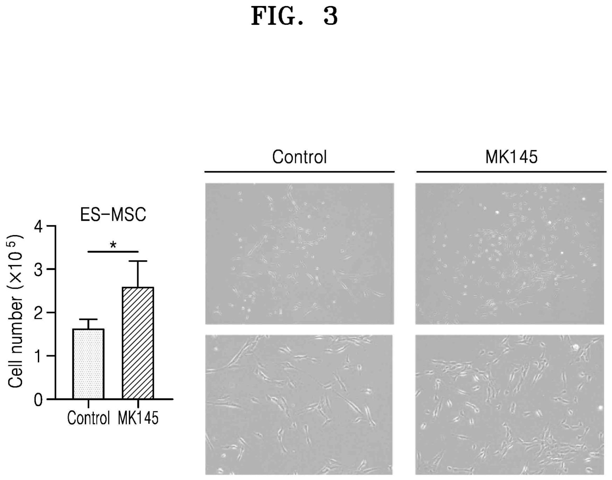

To confirm increased cell proliferation capacity in ES-MSCs transduced with the pET28a-MK145 recombinant plasmid produced by the method of Example 1.1, the number of cells was directly counted. An ES-MSC cell line was used as a control, and the results thereof are shown in FIG. 3.

FIG. 3 confirms an increase in the number of cells in ES-MSCs transduced with a recombinant plasmid vector.

As shown in FIG. 3, cell counting under a microscope confirmed that the number of cells increased by 1.5-fold or more in ES-MSCs transduced with the recombinant plasmid compared to the control.

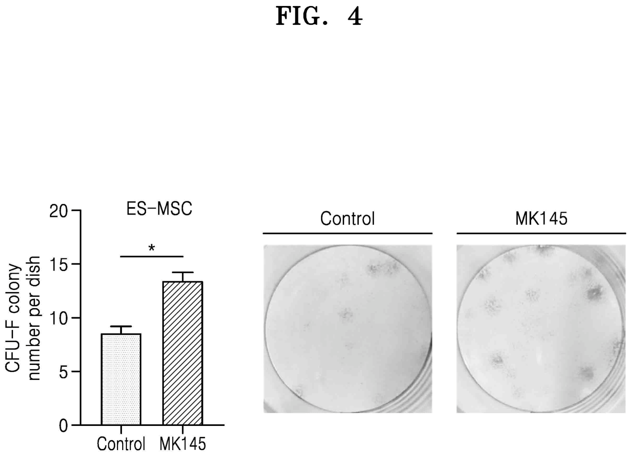

Additionally, the control and ES-MSCs transduced with the recombinant plasmid were seeded at 100 cells per well in 6-well plates and cultured for 2 weeks, and then Crystal Violet staining was performed to confirm the number of cells, and the results thereof are shown in FIG. 4.

FIG. 4 confirms an increase in cell growth in ES-MSCs transduced with a recombinant plasmid vector.

As shown in FIG. 4, it was confirmed that colony formation increased in wells where ES-MSCs transduced with the recombinant plasmid vector were cultured, compared to the control.

### 1.2. Confirmation of increased stemness in ES-MSCs

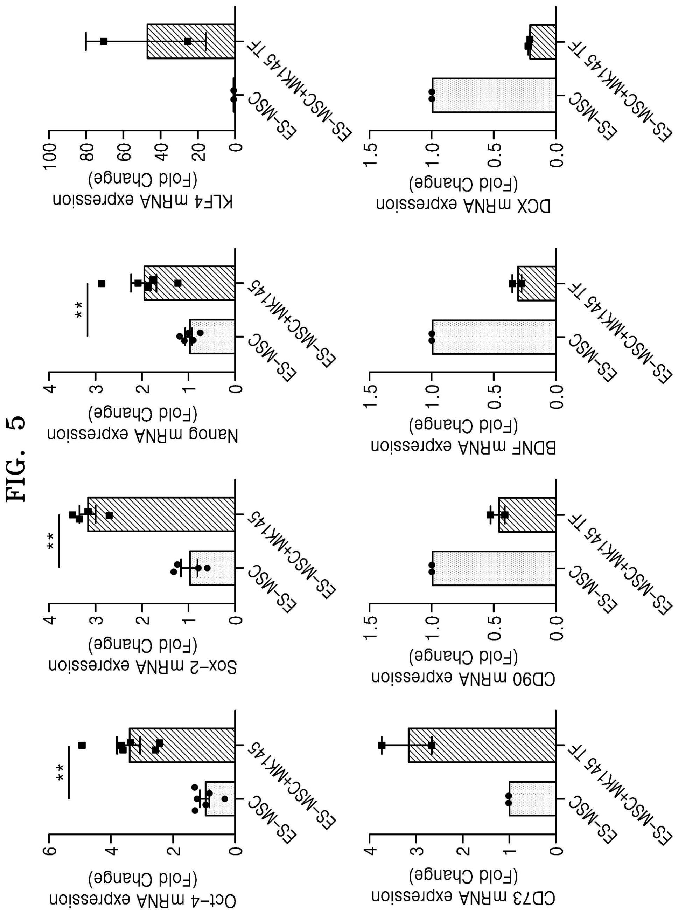

To confirm increased stemness in ES-MSCs transduced with the pET28a-MK145 recombinant plasmid produced by the method of Example 1.1, RNA was extracted, cDNA was synthesized, and RT-PCR was performed. The expression levels of stemness genes Oct4, Sox2, Nanog, and KIf4, which are known as dedifferentiation markers or stemness markers, were examined, and the results thereof are shown in FIG. 5.

FIG. 5 illustrates graphs confirming the expression of stemness markers by RT-PCR in ES-MSCs transduced with a recombinant plasmid vector.

As shown in FIG. 5, it was confirmed that the expression of Oct4, Sox2, Nanog, and Klf4 was significantly increased in ES-MSCs transduced with the recombinant plasmid vector. The expression of DCX, which is a neural marker, and BDNF, which is a brain-derived neurotrophic factor, was reduced, confirming that a dedifferentiated neural state was induced by increased stemness. In addition, CD73, which is an MSC marker, increased and CD90 decreased, confirming that differentiation capacity was enhanced.

Through the above results, it was confirmed that cell proliferation capacity and stemness increased when ES-MSCs were transduced with the recombinant plasmid vector including the nucleotide sequence of SEQ ID NO: 2.

### Experimental Example 2. Confirmation of increased stemness in AD-MSCs transduced with recombinant plasmid

### 2.1. Confirmation of increased cell proliferation capacity in AD-MSCs

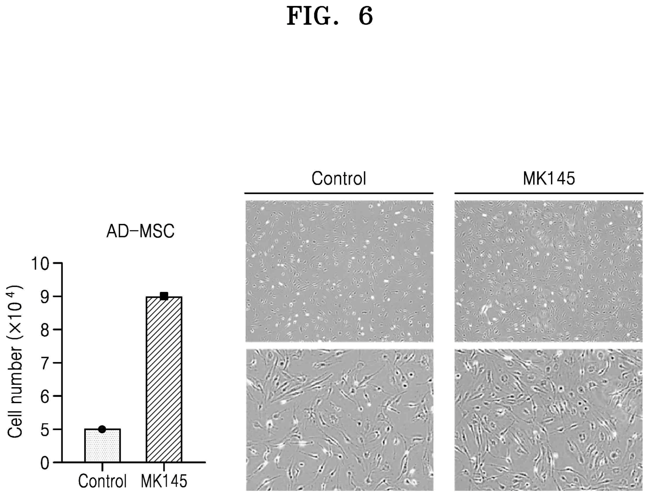

To confirm cell proliferation capacity in AD-MSCs transduced with the pET28a-MK145 recombinant plasmid produced by the method of Example 1.1, the number of the cells was directly counted. An AD-MSC cell line was used as a control, and the results thereof are shown in FIG. 6.

FIG. 6 confirms an increase in the number of cells in AD-MSCs transduced with a recombinant plasmid vector.

As shown in FIG. 6, cell counting under a microscope confirmed that the number of cells increased by 1.5-fold or more in AD-MSCs transduced with the recombinant plasmid vector compared to the control.

### 2.2. Confirmation of increased stemness in AD-MSCs

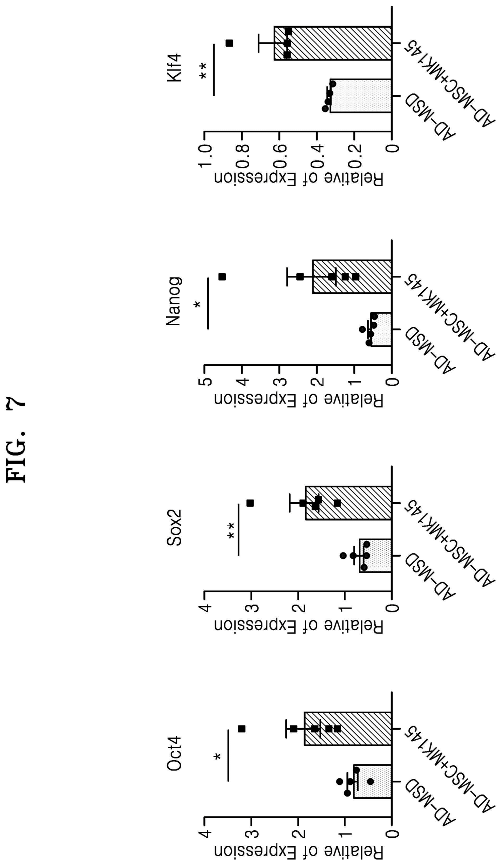

To confirm increased stemness in AD-MSCs transduced with the pET28a-MK145 recombinant plasmid produced by the method of Example 1.1, the cells were harvested, RNA was extracted, cDNA was synthesized, and RT-PCR was performed. The expression levels of stemness genes Oct4, Sox2, Nanog, and Klf4, which are known as dedifferentiation markers or stemness markers, were examined, and the results thereof are shown in FIG. 7.

FIG. 7 illustrates graphs confirming the expression of stemness markers by RT-PCR in AD-MSCs transduced with a recombinant plasmid vector.

As shown in FIG. 7, the expression of Oct4, Sox2, Nanog, and Klf4 was significantly increased in AD-MSCs transduced with the recombinant plasmid vector, confirming increased stemness.

Through the above results, it was confirmed that cell proliferation capacity and stemness increased when AD-MSCs were transduced with the recombinant plasmid vector including the nucleotide sequence of SEQ ID NO: 2.

### Experimental Example 3. Confirmation of increased stemness in fibroblasts transduced with recombinant plasmid

### 3.1. Confirmation of increased cell proliferation capacity in fibroblasts

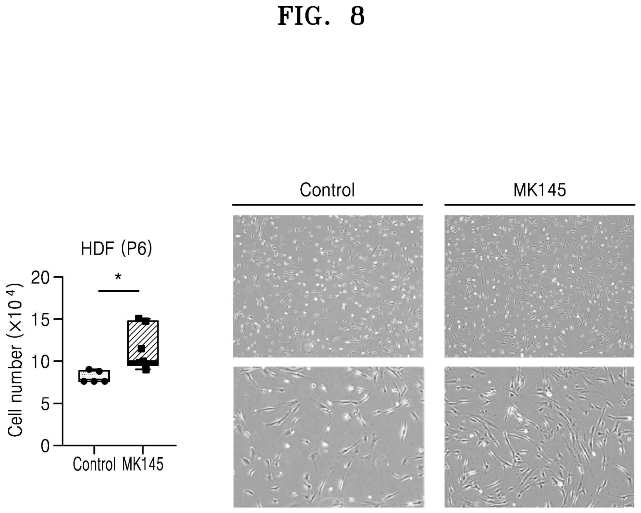

To confirm cell proliferation capacity when the pET28a-MK145 recombinant plasmid was transduced into human dermal fibroblasts (HDFs), the number of cells was directly counted. A fibroblast (HDF) cell line was used as a control, and the results thereof are shown in FIG. 8.

FIG. 8 confirms an increase in the number of cells in a fibroblast cell line transfected with a recombinant plasmid vector.

As shown in FIG. 8, the number of cells increased by approximately 1.5-fold or more in fibroblasts transduced with the recombinant plasmid vector compared to the control, confirming increased cell proliferation capacity.

### 3.2. Confirmation of increased stemness in fibroblasts

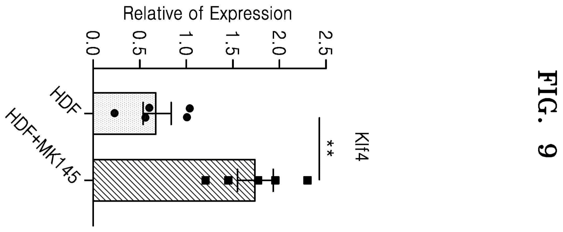

To confirm increased stemness in fibroblasts transduced with the pET28a-MK145 recombinant plasmid produced by the method of Example 1.1, the cells were harvested, RNA was extracted, cDNA was synthesized, and RT-PCR was performed. The expression level of stemness gene KIf4, which is known as a dedifferentiation marker or a stemness marker, was examined, and the results thereof are shown in FIG. 9.

FIG. 9 is a graph confirming the expression of KIf4, which is a stemness marker, in a fibroblast cell line transduced with a recombinant plasmid vector.

As shown in FIG. 9, the expression of KIf4 was significantly increased in fibroblasts transduced with the recombinant plasmid vector, confirming increased stemness.

Through the above results, it was confirmed that cell proliferation capacity and stemness increased when somatic cells were transduced with the recombinant plasmid vector including the nucleotide sequence of SEQ ID NO: 2.

### Experimental Example 4. Evaluation of therapeutic efficacy of factor for inducing stemness in stroke animal model

### 4.1. Establishment of stroke animal model

As a method of blocking blood flow to the middle carotid artery (MCA) by occluding the MCA through insertion of a monofilament thread for suturing via the internal carotid artery (ICA), maintaining the thread results in a permanent model, while removing the thread to induce reperfusion results in a transient occlusion procedure.

Male Sprague-Dawley (SD) rats (200-250 g) aged 6-8 weeks were maintained under a 12-hour light-dark cycle at 21 °C and provided with food and water.

As a stroke animal model, a transient middle cerebral artery occlusion (MCAO) model with reperfusion after MCA occlusion was created. SD rats were anesthetized by inhalation with 3 % isoflurane, and then anesthesia was maintained with 1.5 % isoflurane during surgery using a respiratory anesthesia device. A midline incision was made in the neck area, and the right common carotid artery (CCA), external carotid artery (ECA), and internal carotid artery (ICA) were exposed without damaging the vagus nerve. First, the common carotid artery and the external carotid artery were ligated with black silk sutures, and a small hole was made in the vessel wall of the external carotid artery using microscissors. The prepared 4-0 nylon filament was inserted from the external carotid artery with the small hole through the right internal carotid artery to the middle cerebral artery (1.5-1.8 mm) for occlusion. The skin of the incised neck was closed, and after 90 minutes, the filament inserted into the middle cerebral artery was removed to allow blood reperfusion. Body temperature was maintained at 37 °C using a rectal thermometer during surgery.

### 4.2. Administration of ES-MSCs transduced with factor for inducing stemness to stroke animal model

ES-MSCs transduced with the pET28a-MK145 recombinant plasmid prepared by the method of Example 1.1 (ES-MSC+MK145) were harvested 24 hours after transduction, and 3×10⁶ cells per rat were suspended in 1 ml of physiological saline and injected into rats via tail vein injection.

As a positive control, ES-MSCs that had undergone 12 passages (p12) were used, and the cells were frozen at 5×10⁶ cells per vial. When used, 3×10⁶ cells per rat were suspended in 1 ml of physiological saline and injected via tail vein injection.

For the control, the same amount of physiological saline was injected.

The therapeutic agents (ES-MSC+MK145 and ES-MSC) were administered on day 7 after MCAO model establishment, and all animal model experiments were performed in a blinded manner.

### 4.3 Analysis of behavioral assessments in stroke animal model

Behavioral assessments were performed six times in total on days 1, 3, 7, 14, 21, and 28 after surgery.

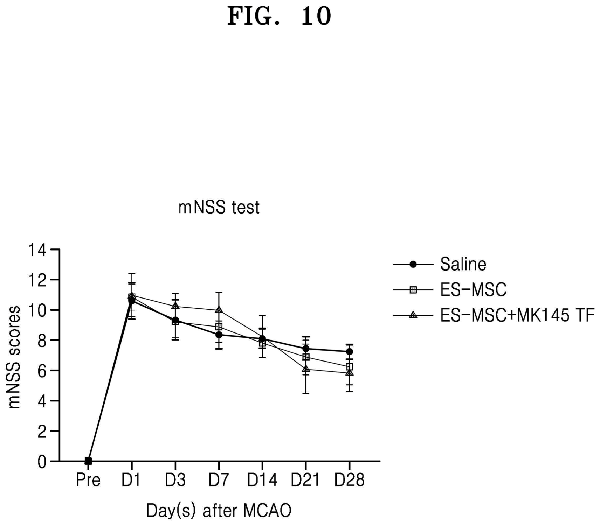

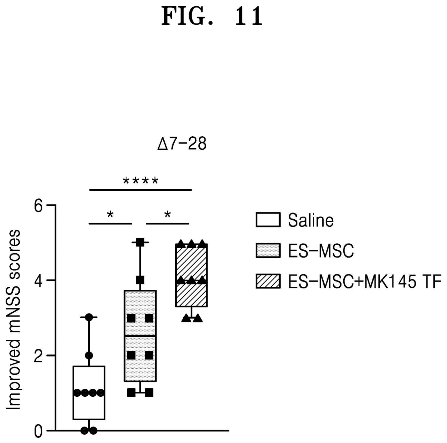

Modified neurological severity scores (mNSS): An analytical method with a maximum score of 14 points, where a score of 0 represents normal behavior and scores closer to 14 indicate more severe neurological deficits. Limb muscle tone (total 3 points), gait (total 3 points), balance ability (total 6 points), and sensory function (total 2 points) were comprehensively evaluated and summed. The evaluation results thereof are shown in FIGS. 10 and 11.

Consequently, the mNSS score on day 28 after surgery was 5.88 in the ES-MSC+MK145 group, which was lower than 6.25 in the ES-MSC group, indicating the most excellent therapeutic effect for stroke. In addition, the difference in behavioral ability scores between day 7 and day 28 after surgery was 4.13 in the ES-MSC+MK145 group, which was greater than 2.63 in the ES-MSC group, indicating a superior therapeutic effect for stroke.

Through this, it was found that treatment with ES-MSC+MK145 transduced with the factor for inducing stemness had a superior therapeutic effect for stroke compared to treatment with MSCs. This indicates that the factor for inducing stemness according to the present disclosure, a vector including the same, and cells including the same can be used as therapeutic agents for stroke.

### 4.3 Analysis of neurogenesis and angiogenesis markers in stroke animal model

On day 28 after administration of MSCs and completion of behavioral assessments, the animal model was sacrificed by blood removal and brain tissues were extracted. The extracted brain tissues were disrupted using lysis beffer, and then centrifuged to isolate proteins. The expression levels of protein markers related to stroke treatment efficacy were examined using 30 µg of the isolated proteins by Western blotting (n=3).

Neurogenesis markers are DCX, Nestin, and NeuN, and an angiogenesis marker is CD31.

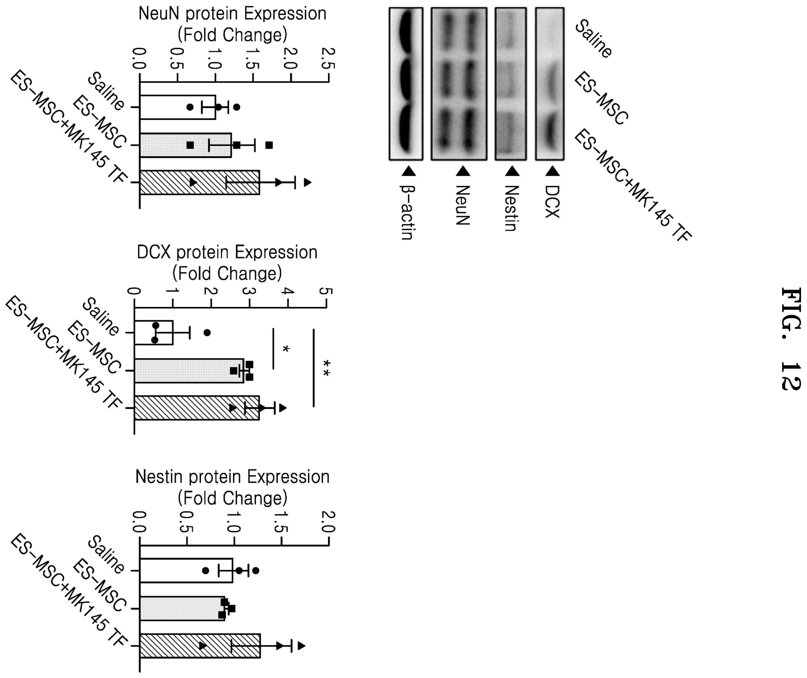

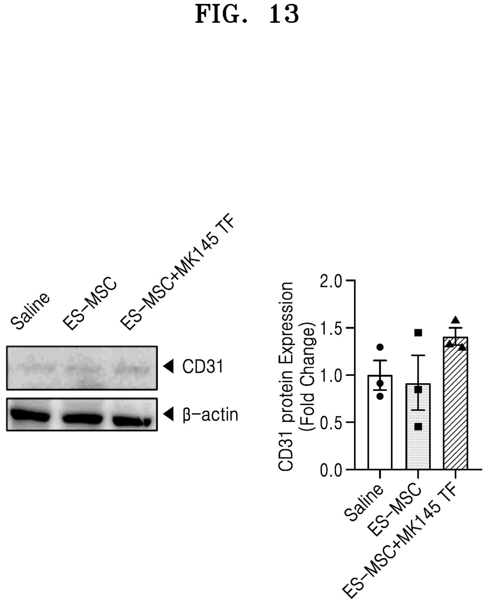

The Western blot results of the neurogenesis markers are shown in FIG. 12, and the Western blot results of the angiogenesis marker are shown in FIG. 13.

As a result, the expression levels of neurogenesis markers DCX, Nestin, and NeuN and the angiogenesis marker CD31 were higher in the ES-MSC+MK145 group than in the ES-MSC group, indicating the most superior therapeutic effect for stroke. In addition, the ES-MSC group showed lower expression levels of the neurogenesis marker NeuN and the angiogenesis marker CD31 than those of the control.

Through this, it was found that treatment with ES-MSC+MK145 transduced with the factor for inducing stemness had a superior therapeutic effect for stroke compared to treatment with MSCs. This indicates that the factor for inducing stemness according to the present disclosure, a vector including the same, and cells including the same can be used as therapeutic agents for stroke.

### Experimental Example 5. Evaluation of therapeutic efficacy of factor for inducing stemness in stroke cell model

### 5.1. Establishment of stroke cell model

An oxygen glucose deprivation model (OGD model), which is a stroke cell model that induces ischemia at the cellular level, was established by inducing ischemia in SH-SY5Y neuroblastoma cells under conditions of 8 % oxygen concentration and glucose-depleted medium, followed by restoration to baseline conditions after 6 hours to induce reperfusion damage.

### 5.2 Administration of ES-MSCs transduced with factor for inducing stemness to stroke cell model

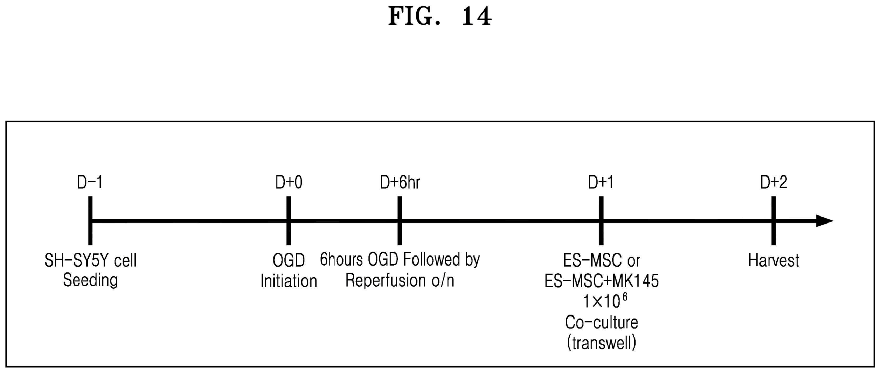

A treatment schedule in the stroke cell model is shown in FIG. 14.

Using insert plates with a 40 µm pore size, SH-SY5Y neuroblastoma cells were cultured in the bottom compartments, and therapeutic stem cells were co-cultured in inserts to confirm therapeutic efficacy.

Specifically, OGD was initiated 1 day after SH-SY5Y cells were seeded. 1 day after OGD initiation, ES-MSCs transduced with the pET28a-MK145 recombinant plasmid produced by the method of Example 1.1 (ES-MSC+MK145) and ES-MSCs were administered to culture media at 1×10⁶ cells, and then harvested 1 day after co-culture to confirm therapeutic efficacy.

### 5.3 Analysis of cell recovery and cell proliferation in stroke cell model

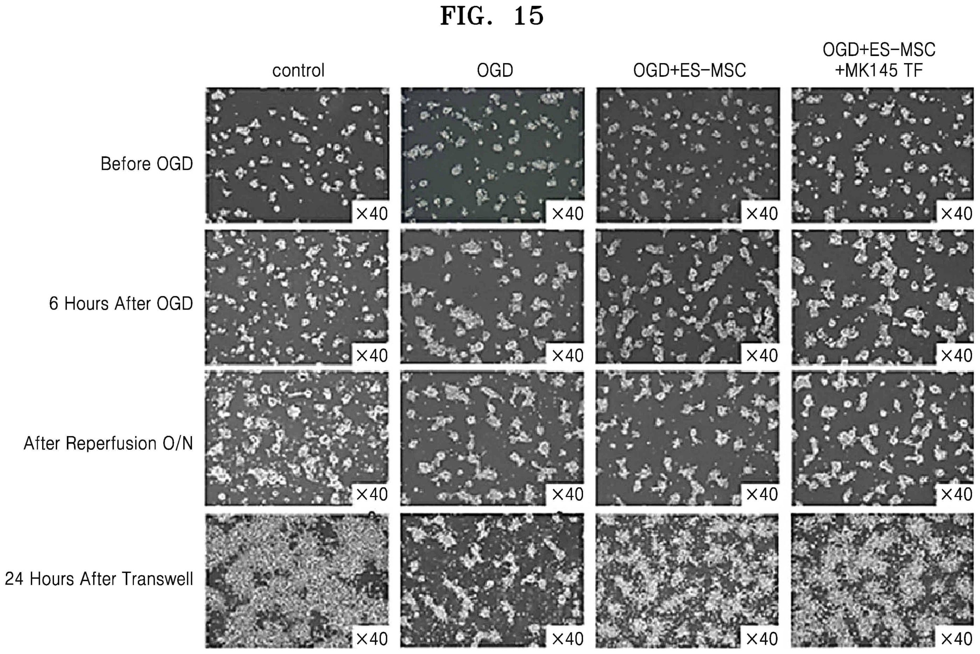

After the ischemic stroke cell model (OGD model) was induced in SH-SY5Y cells, recovery and proliferation of the cells upon treatment with the factor for inducing stemness were analyzed. The results thereof are shown in FIG. 15.

As a result, when ES-MSC+MK145 and the OGD model were co-cultured, cell recovery and proliferation were superior to those when ES-MSCs and the OGD model were co-cultured, indicating that ES-MSC+MK145 showed the most excellent therapeutic effect for stroke.

Through this, it was found that treatment with ES-MSC+MK145 transduced with the factor for inducing stemness had a superior therapeutic effect for stroke compared to treatment with MSCs. This indicates that the factor for inducing stemness according to the present disclosure, a vector including the same, and cells including the same can be used as therapeutic agents for stroke.

### Experimental Example 6. Evaluation of therapeutic efficacy of factor for inducing stemness in dementia animal model

### 6.1. Establishment of amyloid beta injection animal model

Male B6 mice at 11 weeks of age weighing approximately 23-25 g were used, and 5 mice per cage were housed under 12-hour light-dark cycles at 21 °C and provided with food and water.

Amyloid beta1-42, which is a substance that causes memory deficits, was dissolved in 10 % dimethyl sulfoxide (DMSO) saline, incubated at 37 °C for 1 week, and prepared at a concentration of 100 µM.

Experimental animals were anesthetized by inhalation with 3 % isoflurane, and then anesthesia was maintained with 1.5 % isoflurane during surgery using a respiratory anesthesia device. After a stereotaxic frame was prepared, the heads of the experimental animals were fixed, and 4 µl of amyloid beta1-42 was injected via ICV injection using a 10 µL Hamilton microsyringe equipped with a 26-gauge needle at coordinates of -0.9 mm posterior to bregma, 1.7 mm lateral, and 2.2 mm depth over 10 minutes.

### 6.2. Administration of ES-MSCs transduced with factor for inducing stemness to dementia animal model

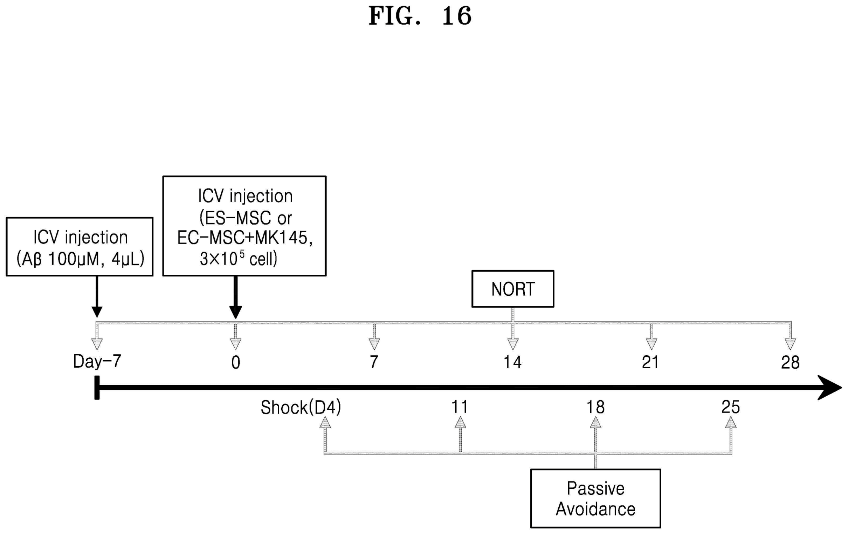

A treatment schedule in the amyloid beta injection animal model is shown in FIG. 16.

Seven days after ICV administration of amyloid beta, ES-MSCs transduced with the pET28a-MK145 recombinant plasmid produced by the method of Example 1.1 (ES-MSC+MK145) and ES-MSCs were administered via ICV injection at 3×10⁵ cells.

### 6.3. Analysis of behavioral/cognitive assessments in amyloid beta injection animal model

Before amyloid beta ICV administration, animals were trained on behavioral/cognitive ability assessments including novel object recognition test (NORT) and passive avoidance test, and scored, followed by evaluation. On day 7 after amyloid beta ICV administration, lesion models were selected and experiments were conducted. On day 7 after amyloid beta ICV administration, therapeutic agents (ES-MSC+MK145 and ES-MSC) were administered, and cognitive function improvement was measured after treatment.

For NORT, measurements were taken after amyloid beta administration, after treatment with the therapeutic agents, and on days 7, 14, 21, and 28 after treatment.

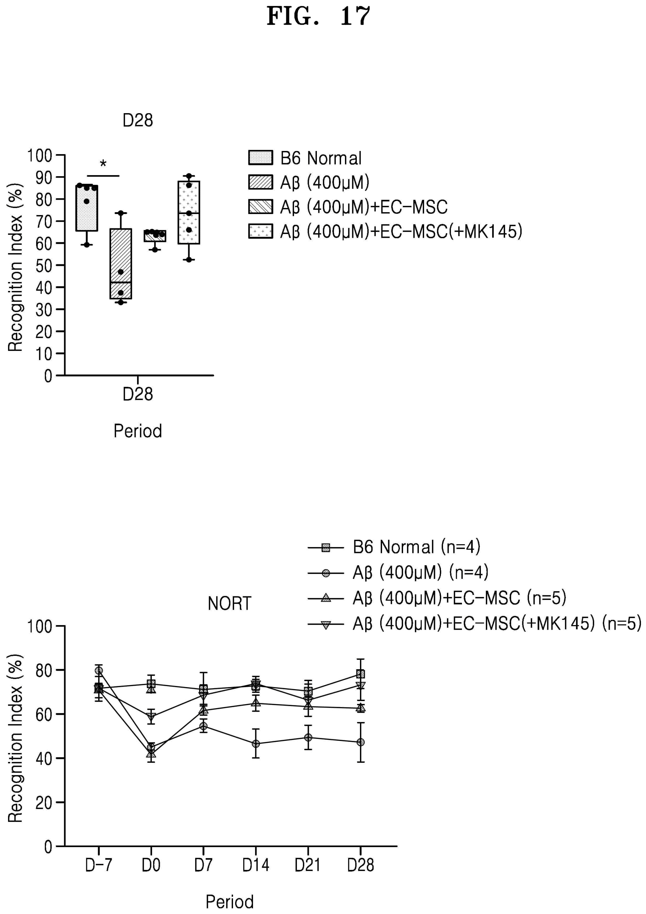

Novel Object Recognition Test (NORT): A cognitive experimental technique that stimulates curiosity in rodents, in which, during the initial habituation training period, memories of two identical objects are instilled, followed by, at the beginning of measurement, replacing one with a novel object of a different color, thereby inducing instinctive curiosity to measure memory. Animals were allowed to freely explore familiar objects for 2 minutes, followed by replacing one with a novel object and taking measurements for 5 minutes. The time spent with the novel object was divided by total time spent exploring both objects, and then expressed as a percentage. The results thereof are shown in FIG. 17.

For the Passive Avoidance Test, an electric shock was applied on day 4 after treatment with the therapeutic agents, and measurements were taken on days 11, 18, and 25 after treatment.

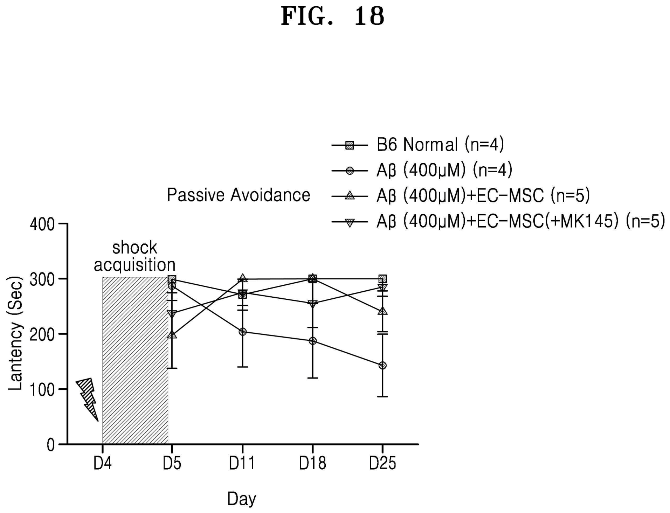

Passive Avoidance Test: One of the representative behavioral experiments for measuring learning and memory ability, which uses the natural tendency of rodents to move toward dark places. The step-through latency, which is the time it takes for experimental animals to, based on their memory, re-enter the same box in which an aversive stimulus (electric shock) had previously been presented, is measured and compared. Longer latency to enter an electrified box indicates better memory. The results thereof are shown in FIG. 18.

As a result, in the novel object recognition test, the ES-MSC+MK145 group showed higher recognition index (RI) values than the ES-MSC group throughout the evaluation, and from day 7 after treatment with the therapeutic agents, exhibited RI values similar to those of normal mice. In the passive avoidance test, in evaluation on day 25 after treatment with the therapeutic agents, the ES-MSC+MK145 group showed higher latency than the ES-MSC group and exhibited latency similar to that of normal mice.

Through this, it was found that treatment with ES-MSC+MK145 transduced with the factor for inducing stemness had a superior therapeutic effect for dementia compared to treatment with MSCs. This indicates that the factor for inducing stemness according to the present disclosure, a vector including the same, and cells including the same can be used as therapeutic agents for dementia.

### Experimental Example 7. Evaluation of therapeutic efficacy of factor for inducing stemness in APP/PS1 TG dementia animal model

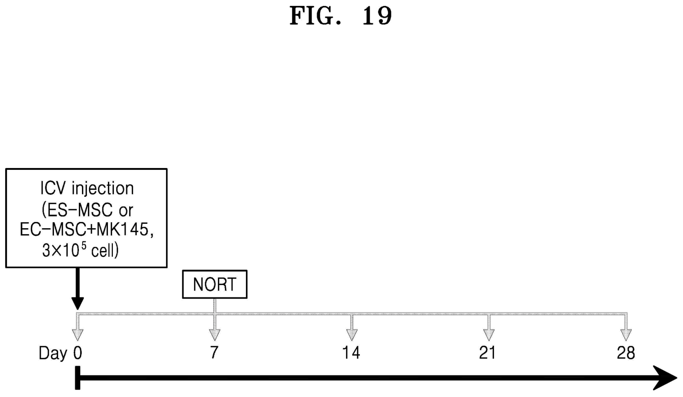

ES-MSCs transduced with the pET28a-MK145 recombinant plasmid produced by the method of Example 1.1 (ES-MSC+MK145) or ES-MSCs were administered to a 10-month-old APP/PS1 TG animal model via ICV injection at 3×10⁵ cells. A treatment schedule in the APP/PS1 TG dementia animal model is shown in FIG. 19.

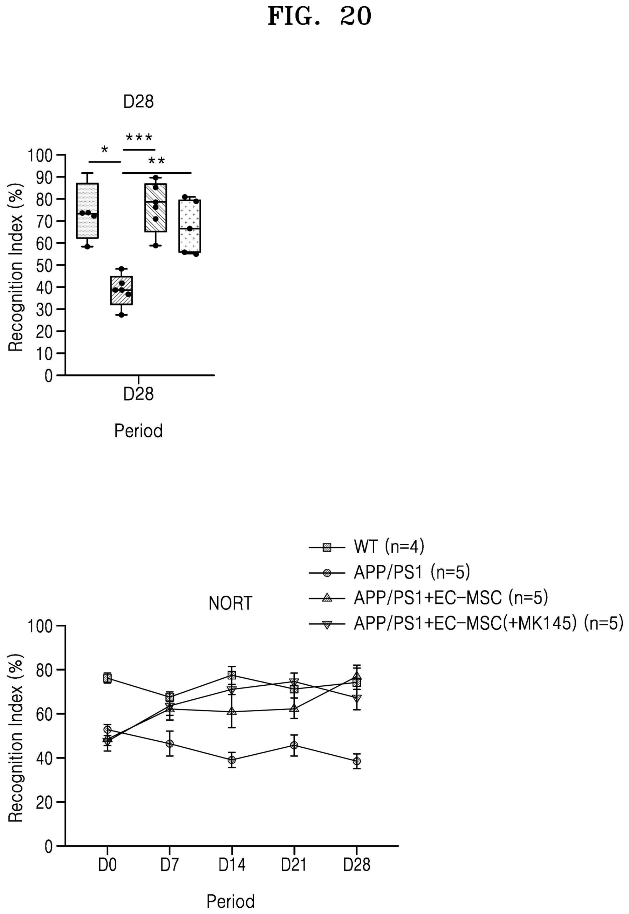

Animals were trained on behavioral/cognitive ability assessments using the novel object recognition test (NORT) and scored, followed by evaluation. Upon treatment with the therapeutic agents (ES-MSC+MK145 and ES-MSC), improvement in cognitive function was measured. For NORT, measurements were taken after treatment with the therapeutic agents, and on days 7, 14, 21, and 28 after treatment. The results thereof are shown in FIG. 20.

As a result, in the novel object recognition test, the ES-MSC+MK145 group and the MSC+MK145 group showed higher RI values than the untreated APP/PS1 TG dementia animal group throughout the evaluation, and in evaluation on day 28 after treatment with the therapeutic agents, the ES-MSC+MK145 group and the ES-MSC group exhibited RI values similar to those of normal mice.

Through this, it was found that treatment with ES-MSC+MK145 transduced with the factor for inducing stemness had a superior therapeutic effect for dementia compared to treatment with MSCs. This indicates that the factor for inducing stemness according to the present disclosure, a vector including the same, and cells including the same can be used as therapeutic agents for dementia.

From the foregoing description, it will be understood by those of ordinary skill in the art to which the present disclosure pertains that the present disclosure may be embodied in other particular forms without changing the technical spirit or essential characteristics of the present disclosure. Therefore, the above-described experimental examples and examples should be construed as being provided for illustrative purposes only and not for purposes of limitation. The scope of the present disclosure should be construed as including all modifications and modified forms derived from the meaning and scope of the appended claims and the concept of equivalents thereto rather than the detailed description.

## Claims

1. A factor for inducing stemness in cells, the factor comprising a nucleotide sequence encoding an amino acid sequence having at least 90 % sequence identity with the amino acid sequence of SEQ ID NO: 1.

2. The factor for inducing stemness of claim 1, wherein
the cells are somatic cells, lineage-restricted progenitor cells, or stem cells.

3. The factor for inducing stemness of claim 2, wherein
the somatic cells are fibroblasts, epithelial cells, muscle cells, cardiac cells, kidney cells, neurons, hair cells, hair root cells, hair follicle cells, oral epithelial cells, beta cells, gastric mucosal cells, goblet cells, G cells, B cells, pericytes, hyalocytes, odontoblasts, chondrocytes, or cells extracted from urine, saliva, sweat, or blood from the human body.

4. The factor for inducing stemness of claim 2, wherein
the lineage-restricted progenitor cells are any one selected from the group consisting of neural progenitor cells, glial progenitor cells, blood endothelial progenitor cells, mesenchymal progenitor cells, hematopoietic progenitor cells, pancreatic progenitor cells, myeloid progenitor cells, and lymphoid progenitor cells.

5. The factor for inducing stemness of claim 2, wherein
the stem cells are embryonic stem cells, adult stem cells, or induced pluripotent stem cells.

6. The factor for inducing stemness of claim 5, wherein
the adult stem cells are derived from one or more tissues selected from the group consisting of umbilical cord blood, adipose, nerve, bone marrow, peripheral blood, muscle, liver, skin, placenta, amniotic membrane, embryo, and umbilical cord.

7. The factor for inducing stemness of claim 5, wherein
the adult stem cells are one or more selected from the group consisting of hematopoietic stem cells (HSCs), mesenchymal stem cells (MSCs), neural stem cells (NSCs), cardiac stem cells (CSCs), intestinal stem cells (ISCs), muscle stem cells (MuSCs), follicle stem cells (FSCs), and endothelial progenitor cells (EPCs).

8. The factor for inducing stemness of claim 7, wherein
the mesenchymal stem cells (MSCs) are one or more selected from the group consisting of embryo, bone marrow, adipose, umbilical cord blood, synovial membrane, trabecular bone, muscle, nerve, and infrapatellar fat pad.

9. A composition for increasing or maintaining stemness in cells, the
composition comprising one or more selected from the group consisting of: the factor for inducing stemness in cells of any one of claims 1 to 8; a vector comprising the factor; and a protein of the factor.

10. The composition of claim 9, wherein
the vector is one or more vectors selected from the group consisting of a plasmid vector, a cosmid vector, a viral vector, a lentivirus vector, a retrovirus vector, a human immunodeficiency virus (HIV) vector, a murine leukemia virus (MLV) vector, an avian sarcoma/leukosis (ASLV) vector, a spleen necrosis virus (SNV) vector, a Rous sarcoma virus (RSV) vector, a mouse mammary tumor virus (MMTV) vector, an adenovirus vector, an adeno-associated virus vector, a Herpes simplex virus vector, and an episomal vector.

11. The composition of claim 9, wherein
the composition causes: increased proliferation capacity of cells; induction of dedifferentiation of cells; or both.

12. The composition of claim 9, wherein
the composition increases the expression or activity of one or more selected from the group consisting of Nanog, octamer-binding transcription factor 4 (OCT4), sex determining region Y (SRY)-box 2 (SOX-2), and Kruppel-like factor 4 (Klf4).

13. A method of producing cells having increased or maintained stemness, the method comprising treating cells with the composition of claim 9.

14. The method of claim 13, wherein dedifferentiation of cells is induced by the increased stemness.

15. A gene therapeutic composition for the prevention or treatment of a neurological disease, comprising one or more selected from the group consisting of: the factor for inducing stemness in cells of any one of claims 1 to 8; and a vector comprising the factor.

16. The gene therapeutic composition of claim 15, wherein the neurological disease is one or more selected from the group consisting of ischemic stroke, hemorrhagic stroke, ischemic brain injury, traumatic brain injury, cerebral palsy, mental disorder, and neurodegenerative disease.

17. A cell therapeutic composition for the prevention or treatment of a neurological disease, comprising cells including one or more selected from the group consisting of: the factor for inducing stemness in cells of any one of claims 1 to 8; a vector comprising the factor; and a protein of the factor.

18. The cell therapeutic composition of claim 17, wherein the neurological disease is one or more selected from the group consisting of ischemic stroke, hemorrhagic stroke, ischemic brain injury, traumatic brain injury, cerebral palsy, mental disorder, and neurodegenerative disease.
